# EUROPEAN PATENT SPECIFICATION

(11) **EP 2 761 995 B1**
(45) Date of publication and mention of the grant of the patent: **02.11.2016**
(21) Application number: 14153619.3
(22) Date of filing: 03.02.2014
(51) Int. Cl.: A01G 1/00, A01G 7/04, A01G 31/00, A01G 33/00, A01H 3/02

(54) **Plant cultivation lamp and plant cultivation method using the same**
Pflanzenzuchtlampe und Pflanzenzuchtverfahren damit
Lampe de culture de plantes et procédé de culture de plantes l'utilisant

(30) Priority: 04.02.2013 JP 2013019930; 31.01.2014 JP 2014017586
(43) Date of publication of application: 06.08.2014
(73) Proprietor: SHOWA DENKO K.K., Tokyo 105-8518 (JP)
(72) Inventor: Takeuchi, Ryouichi, Tokyo, 105-8518 (JP); Ara, Hironori, Tokyo, 105-8518 (JP); Suzuki, Hiroshi, Tokyo, 105-8518 (JP)
(74) Representative: Strehl Schübel-Hopf & Partner

(56) References cited:
- EP-A1- 2 462 797
- JP-A- H08 103 167
- US-A1- 2012 043 907
- US-A1- 2012 293 993

## Description

### BACKGROUND OF THE INVENTION

### Field of the Invention

The present invention relates to a plant cultivation lamp and a plant cultivation method using the same. More specifically, the present invention relates to a plant cultivation lamp that promotes growth of a plant by irradiating the plant with artificial light and a plant cultivation method using the same.

Priority is claimed on Japanese Patent Application No. 2013-019930, filed on February 4, 2013 and Japanese Patent Application No.2014-017586, filed on January 31, 2014.

### Description of Related Art

In the related art, there is a technology that promotes raising seedlings by irradiating the plant seedlings with artificial light in plant cultivation. It is possible to shorten a cultivation period and to increase the number of harvesting times at the same place by promoting growth of a plant. In addition, it is possible to increase the amount of harvested crop if the plant can grow bigger under the same cultivation period conditions.

As a method of plant cultivation using artificial light irradiation, for example, Japanese Unexamined Patent Application, First Publication No.H6-276858 discloses an apparatus that irradiates a plant with light configured to alternatingly irradiate the plant with green light and white light. The irradiation apparatus is configured to switch conditions between day and night by alternatingly irradiating a plant with green light having a wavelength range between 500 nm and 570 nm and white light having a wavelength range between 300 nm and 800 nm and grows the plant by facilitating an operation of translocation of a plant.

In addition, for example, Japanese Unexamined Patent Application, First Publication No.H8-103167 discloses a light source for plant cultivation that irradiates a plant with light energy to culture, grow, cultivate, and tissue-culture a plant by simultaneously or alternatingly turning on a light emitting diode that emits blue light (400 nm to 480 nm) and a light emitting diode that emits red light (620 nm to 700 nm). The light source for plant cultivation is designed to cultivate a plant with good energy efficiency by irradiating a plant only with light having a wavelength that corresponds to a light-absorption peak of chlorophyll (near 450 nm and near 660 nm).

Japanese Unexamined Patent Application, First Publication No.H8-103167 defines that the plant may be simultaneously or alternatingly irradiated with the blue light and the red light (refer to [Claim 1] of the document). However, Japanese Unexamined Patent Application, First Publication No.H8-103167 confirms healthy growth (compared to unhealthy growth such as succulent growth in independent irradiation) under a cultivation condition of simultaneous irradiation with the blue light and the red light similarly to a cultivation condition in the sunlight, in comparison among independent irradiation with blue light, independent irradiation with red light, and simultaneous irradiation of blue light and red light (refer to paragraph [0011] of the document). In addition, the alternating irradiation with the blue light and the red light indicates flashing irradiation with a high frequency (refer to paragraph [0006] of the document) and Japanese Unexamined Patent Application, First Publication No.H8-103167 does not confirm any effect for promoting the growth in a case of alternating irradiation with the blue light and the red light. Accordingly, Japanese Unexamined Patent Application, First Publication No.H8-103167 does not substantially disclose a plant cultivation method using alternating irradiation of the blue light and the red light. US-A1-2012/023993 discloses a plant cultivation lamp with red and blue light emitting elements that can be controlled to give a varying mix of wavelengths.

### SUMMARY OF THE INVENTION

There is a plant cultivation method that includes a step of independently performing a sequence of irradiating a plant with red light and a sequence of irradiating a plant with blue light within a certain period of time (hereinafter, referred to as "Shigyo method").

As a plant cultivation apparatus which is used when performing the Shigyo method, it is conceivable to install means of irradiating red light and means of irradiating blue light to a plant to be grown.

However, in such a plant cultivation apparatus, a space used to dispose two types of irradiation means, a means for irradiating red light and a means for irradiating blue light, is required.

In addition, if there is a deviation between the irradiation direction of red light for a plant and the irradiation direction of blue light for a plant, it is sometimes impossible to obtain a sufficient effect of promoting the growth of the plant in some cases even if the Shigyo method is implemented.

In addition, depending on the type of plant to be cultivated, some plants prefer red light more than blue light. As a method of effectively promoting the growth of such plants, it is conceivable to make the intensity of the red light irradiated to a plant stronger than the intensity of blue light irradiated to a plant. However, it is complicated to change the intensity ratio of red light to blue light with which a plant is irradiated in the plant cultivation apparatus.

The present invention is used when performing the Shigyo method and is designed to solve the above-described problems. An object of the present invention is to provide a plant cultivation lamp that can simultaneously or alternatingly irradiate a plant with red light and blue light; can easily secure a space used to dispose irradiation means; has no deviation between the direction from which a plant is irradiated with red light and the direction from which a plant is irradiated with blue light; can easily change the intensity ratio of red light to blue light; and has an excellent effect for promoting growth of a plant, and a plant cultivation method using the same.

The present invention relates to the following.
(1) According to an aspect of the present invention, there is provided a plant cultivation lamp used in a plant cultivation method including a step of independently performing a sequence of irradiating a plant with red light and a sequence of irradiating the plant with blue light within a certain period of time, including: a light irradiation unit that includes one or more red light emitting elements that emit red light and one or more blue light emitting elements that emit blue light; and a control unit that controls the light irradiation unit to independently turn on and off the red light emitting elements and the blue light emitting elements.
(2) In the aspect stated in the above (1), the light irradiation unit may include mixed color light emitting elements where the red light emitting elements and the blue light emitting elements are equipped with an identical package.
(3) In the aspect stated in the above (1) or (2), in which the light emission intensity ratio of the red light emitting element to the blue light emitting element may be in a range between 2:1 and 9:1.
(4) In the aspect stated in the above any one of (1) to (3), the light irradiation unit may include plural identical red light emitting elements and plural identical blue light emitting elements, wherein the ratio of the number of the red light emitting elements and the number of the blue light emitting elements may be in the range of 2:1 to 9:1, wherein the red light emitting elements and the blue light emitting elements may be arranged to be regularly mixed.
   In addition, in the aspect, the red light emitting elements and the blue light emitting elements may be arranged to be regularly mixed in each of one or more straight lines, in each of which the red light emitting elements and the blue light emitting elements are arranged according to the ratio of the number.
   In addition, in the aspect, the red light emitting elements and the blue light emitting elements may be arranged respectively in separate lines, in which the lines of the red light emitting elements and the lines of the blue light emitting elements are regularly arranged according to the ratio of the number of the red light emitting elements and the number of the blue light emitting elements.
(5) In the aspect stated in the above any one of (1) to (4), the control unit may include light emission intensity control means for controlling the light emission intensity ratio of red light to blue light emitted from the light irradiation unit.
(6) In the aspect stated in the above any one of (1) to (5), the light irradiation unit may have a long rectangular shape in planar view and may have an outer shape compatible with a customized straight tube fluorescent lamp.
(7) In the aspect stated in the above any one of (1) to (6), the light irradiation unit may include red conversion means for red which is electrically connected to the red light emitting elements and conversion means for blue which is electrically connected to the blue light emitting elements, in which the red conversion means may output DC power converted from AC power which is input and may have a function to control an output electric current which is supplied to the red light emitting element, and in which the conversion means for blue may output DC power converted from AC power which is input and may have a function to control an output electric current which is supplied to the blue light emitting elements.
(8) In the aspect stated in the above (7), the light irradiation unit may have a long rectangular shape in planar view and may include two input terminals for the red light emitting elements electrically connected to conversion means for red in one end and two input terminals for the blue light emitting elements electrically connected to conversion means for blue in the other end.
(9) According to an aspect of the present invention, there is provided a plant cultivation method using the aspect stated in the above any one of (1) to (8), including: a sequence of irradiating a plant with red light; and a sequence of irradiating a plant with blue light, in which the sequences are independently performed within a certain period of time.

In the present invention, at least leafy vegetables, fruit trees, grains, and algae are included in the "plant". In addition, phytoplankton such as green algae or mosses is also widely included in the "plant" referred in the present invention.

The plant cultivation lamp of an aspect of the present invention includes the control unit that independently turns on and off the red light emitting elements and the blue light emitting elements. Therefore, it is possible to simultaneously or alternatingly irradiate a plant with red light and blue light depending on the plant to be grown so as to obtain a sufficient effect for promoting the growth of the plant using the Shigyo method, thereby obtaining an excellent effect of promoting the growth of the plant.

In addition, the plant cultivation lamp of an aspect of the present invention includes the light irradiation unit having the red light emitting elements and the blue light emitting elements. Therefore, it is possible to easily secure a space used to dispose the irradiation means and to reduce the deviation between the direction from which a plant is irradiated with red light and the direction from which a plant is irradiated with blue light compared to a case where two types of irradiation means, a means for irradiating red light and a means for irradiating blue light, are disposed in the lamp.

In addition, in a case where the control unit of the plant cultivation lamp of an aspect of the present invention includes the light emission intensity control means used to control the light emission intensity ratio of red light to blue light emitted from the light irradiation unit, it is possible to easily change the intensity ratio of red light to blue light, thereby easily providing an optimum intensity ratio to a plant to be grown.

### BRIEF DESCRIPTION OF THE DRAWINGS

FIG. 1 is a schematic diagram showing an example of a plant cultivation lamp of the present invention.
FIGS. 2A to 2D are plan views showing other examples of the disposition of red light emitting elements and blue light emitting elements in a light irradiation unit of a plant cultivation lamp.
FIGS. 3A to 3c are plan views showing other examples of the disposition of red light emitting elements and blue light emitting elements in a light irradiation unit of a plant cultivation lamp.
FIGS. 4A to 4c are plan views showing other examples of the disposition of red light emitting elements and blue light emitting elements in a light irradiation unit of a plant cultivation lamp.
FIGS. 5A to 5c are plan views showing other examples of the disposition of red light emitting elements and blue light emitting elements in a light irradiation unit of a plant cultivation lamp.

### DETAILED DESCRIPTION OF THE INVENTION

Hereinafter, a preferable embodiment of the present invention is described in reference to the drawings. Note that the embodiment described below illustrates an example of a typical embodiment of the present invention and this does not limit the scope of the present invention.

### [Plant cultivation lamp]

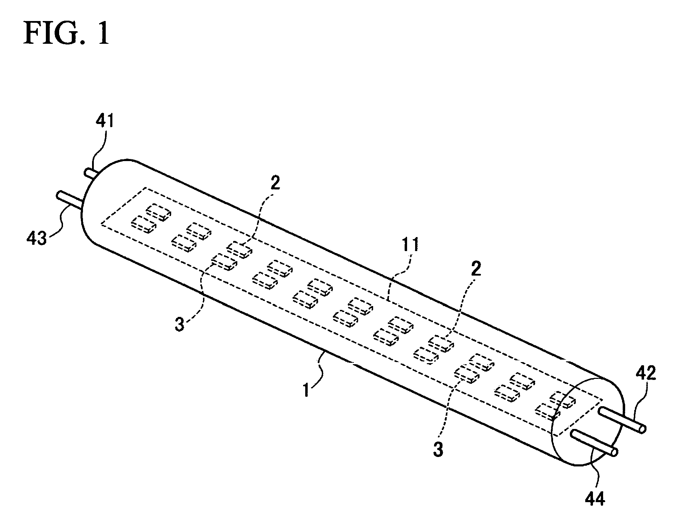

FIG. 1 is a schematic diagram for illustrating an example of a plant cultivation lamp of the present invention. The plant cultivation lamp 1 shown in FIG. 1 is used in a plant cultivation method (Shigyo method) including a step of independently performing a sequence (process) of irradiating a plant with red light and a sequence (process) of irradiating a plant with blue light in fixed time periods (within a certain period of time), and the plant cultivation lamp includes a light irradiation unit 11 and a control unit (not shown) used to control the light irradiation unit.

In the sequence of irradiating a plant with red light of the present invention, irradiation light may include red light, and if the intensity of the red light included in the irradiation light is greater than or equal to 60%, the irradiation light may include light other than red light, such as, for example, blue light. According to the review of the inventor of the present application, the process of irradiating a plant with red light in the Shigyo method allows mixing of the red light with about 30% of blue light in an intensity ratio, and thus it is possible to obtain an effect of enhancing growth of a plant within the allowable range. In order to enhance the effect of the Shigyo method, the mixed amount of blue light is more preferably set to be less than or equal to 20% and most preferably set to be 0%. An example of the irradiation light intensity ratio in the sequence of irradiating a plant with red light includes 60% of red light, 20% of far infrared light, and 20% of blue light, and the most preferable intensity ratio is 100% of red light.

The intensity ratio of the irradiation light in the present invention is obtained using the photosynthetic photon flux density (PPFD, unit: µmol/m²s).

In the sequence of irradiating a plant with blue light of the present invention, irradiation light may include blue light, and if the intensity of the blue light included in the irradiation light is greater than or equal to 60%, the irradiation light may include light other than blue light, such as, for example, red light. According to the review of the inventor of the present application, the process of irradiating a plant with blue light in the Shigyo method allows mixing of the blue light with 30% of red light based on intensity and it is possible to obtain an effect of promoting growth of a plant within the allowable range. In order to enhance the effect of the Shigyo method, the mixed amount of red light is more preferably set to be less than or equal to 20% and most preferably set to be 0%. An example of the irradiation light intensity ratio in the sequence of irradiating a plant with blue light includes 60% of blue light, 20% of far infrared light, and 20% of red light, and the most preferable intensity ratio is 100% of blue light.

As shown in FIG. 1, the light irradiation unit 11 has a long rectangular shape in planar view and has an outer shape compatible with a customized straight tube fluorescent lamp. In addition, the light irradiation unit 11 has a plurality of red light emitting elements 2 that emit red light and a plurality of blue light emitting elements 3 that emit blue light. In the plant cultivation lamp 1 shown in FIG. 1, the number of red light emitting elements 2 is the same as the number of blue light emitting elements 3. The plurality of red light emitting elements 2 and the plurality of blue light emitting elements 3 are each arranged in a line at regular intervals along the longitudinal direction of the light irradiation unit 11. The linearly arranged plurality of red light emitting elements 2 and the plurality of blue light emitting elements 3 are disposed approximately in parallel to each other.

The number of red light emitting elements 2 and the number of blue light emitting elements 3 in the plant cultivation lamp 1 shown in FIG. 1 are the same, but they may be different from each other. Depending on the type of plant to be cultivated, growth of a plant is promoted by making the light emission intensity of red light higher than the light emission intensity of blue light in some cases. In a case of cultivating such a plant, it is preferable to use a plant cultivation lamp 1 where the number of red light emitting elements 2 is increased more than the number of blue light emitting elements 3 included in the light irradiation unit 11. It is possible to make the light emission intensity of red light higher than the light emission intensity of blue light in emitted light from the light irradiation unit 11 by making the number of red light emitting elements 2 higher than the number of blue light emitting elements 3.

A light irradiation unit in which the light emission intensity of red light and the light emission intensity of blue light are different can be made by adjusting any one of the number of the light emitting element and the light emission intensity of the light emitting element. For example, it can be made by making the number of the red light emitting element and the number of the blue light emitting element the same and making the light emission intensity of the red light emitting element and the light emission intensity of the blue light emitting element different from each other. It can be made by making the ratio of the number of identical red light emitting elements and the number of identical blue light emitting elements corresponding to the ratio of the light emission intensities of red light and blue light.

A light irradiation unit in which the light emission intensity of red light and the light emission intensity of blue light are different can also be made by adjusting both of the number of the light emitting element and the light emission intensity of the light emitting element.

In a case where the number of red light emitting elements 2 and the number of the blue light emitting elements 3 are different from each other, the light emission intensity ratio of the red light emitting element 2 to the blue light emitting element 3 (total intensity of red light : total intensity of blue light) is preferably within a range between 2:1 to 9:1, and more preferably within a range between 2:1 to 5:1. When using such a plant cultivation lamp 1, in the emitted light from the light irradiation unit 11 which is obtained when an electric current suitable for light emitting elements of each color is supplied to every red light emitting element 2 and every blue light emitting element 3, the light emission intensity of blue light is sufficiently higher than the light emission intensity of red light. Accordingly, the light emission intensity of red light in the emitted light from the light irradiation unit 11 can be easily made to be sufficiently higher than the light emission intensity of blue light, which is preferable. In addition, the electric current value can be easily adjusted when making the value of the intensity ratio of the red light to the blue light suitable for plant cultivation.

In a case where the light emission intensity ratio of the red light emitting element 2 to the blue light emitting element 3 is lower than the above-described range (in a case where the light emission intensity of blue light is too high), there is a concern that it is impossible to obtain a sufficient effect of promoting the growth of a plant, the effect being caused by making the light emission intensity of red light higher than the light emission intensity of blue light. In a case where the light emission intensity ratio of the red light emitting element 2 to the blue light emitting element 3 is higher than the above-described range, there is a concern that it is impossible to obtain a sufficient effect for promoting the growth of a plant because the light emission intensity of red light is too high.

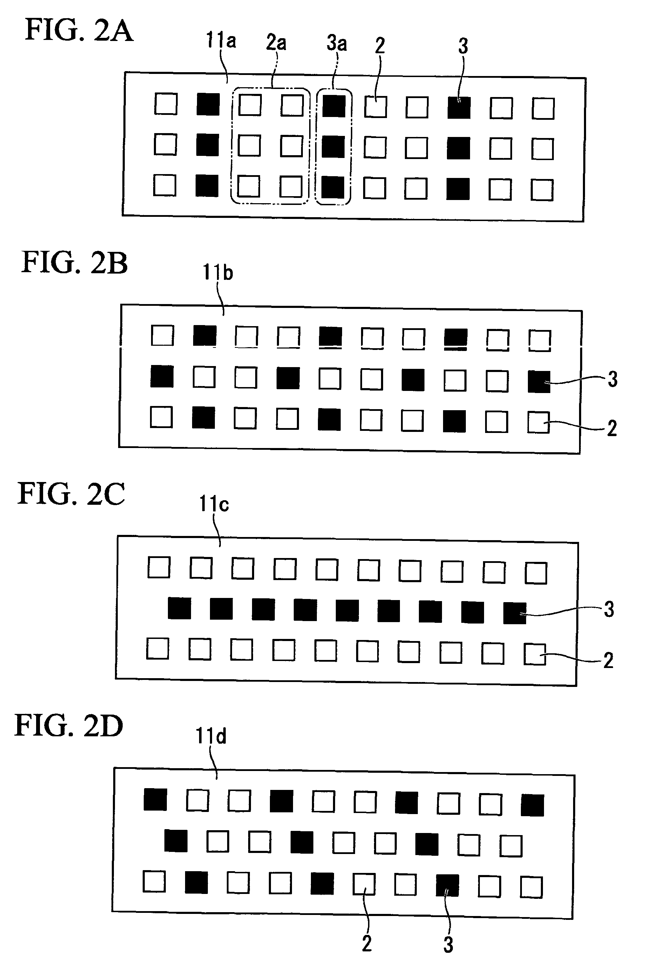

In a case where the number of red light emitting elements 2 and the number of blue light emitting elements 3 included in light irradiation unit 11 are different from each other, it is possible to dispose the red light emitting elements 2 and the blue light emitting elements 3 of the light irradiation unit 11 as shown in FIGS. 2A to 2D instead of disposing the elements as shown in FIG. 1, for example.

FIGS 2A to 2D are plan views showing other examples of disposition of red light emitting elements 2 and blue light emitting elements 3 in a light irradiation unit of a plant cultivation lamp, each of which shows a part of the light irradiation unit. In the light irradiation unit shown in FIGS. 2A to 2D, the ratio of the number of red light emitting elements 2 to the number of blue light emitting elements 3 is set as 2:1. In FIGS. 2A to 2D, the symbol □ indicates red light emitting elements 2 and the symbol ■ indicates blue light emitting elements 3. In the disposition of the red light emitting elements 2 and the blue light emitting elements 3 shown in FIGS. 2A to 2D, irregularity of intensity distribution of emission rarely occurs in a light emission surface, which is preferable.

In the light irradiation unit 11a shown in FIG. 2A, there is a row along a direction orthogonal to the longitudinal direction of the light irradiation unit 11a and a line along the longitudinal direction of the light irradiation unit 11a which are formed by the light emitting elements configured to have the plurality of blue light emitting elements 3 and the plurality of red light emitting elements 2. The intervals between the light emitting elements adjacent to each other are regularly formed. All the lines configured to have the light emitting elements are linearly arranged with a plurality of units formed of a blue light emitting element 3 and two red light emitting elements 2. The rows configured to have the light emitting elements are formed with a blue element group 3a in which a plurality of blue light emitting elements 3 (three in the example shown in FIG. 2A) are linearly arranged in a row and a red element group 2a in which a plurality of red light emitting elements 2 (three in the example shown in FIG. 2A) are linearly arranged in two rows. As shown in FIG. 2A, the red element group 2a and the blue element group 3a are alternatingly arranged in the longitudinal direction of the light irradiation unit 11a.

In the light irradiation unit 11b shown in FIG. 2B, similarly to the light irradiation unit 11a shown in FIG. 2A, there is a row along a direction orthogonal to the longitudinal direction of the light irradiation unit 11b and a line along the longitudinal direction of the light irradiation unit 11b which are formed by the light emitting elements configured to have the plurality of blue light emitting elements 3 and the plurality of red light emitting elements 2. The intervals between the light emitting elements adjacent to each other are regularly formed. Similarly to the light irradiation unit 11a shown in FIG. 2A, a plurality of units formed of a blue light emitting element 3 and two red light emitting elements 2 are arranged in a row. The rows configured to have the light emitting elements shown in FIG. 2B include a row linearly arranged with a blue light emitting element 3 between two red light emitting elements 2, a row linearly arranged with a red light emitting element 2 between two blue light emitting elements 3, and a row linearly arranged with three red light emitting elements 2, and these are arranged in the longitudinal direction of the light irradiation unit 11b in this order.

In the light irradiation unit 11c shown in FIG. 2C, there are three lines formed along the longitudinal direction of the light irradiation unit 11c by the light emitting elements configured to have the plurality of blue light emitting elements 3 and the plurality of red light emitting elements 2. In the light irradiation unit 11c shown in FIG. 2C, a line in which a plurality of blue light emitting elements 3 are linearly arranged between two lines in which a plurality of red light emitting elements 2 are linearly arranged is disposed. The intervals between the light emitting elements adjacent to each other are regularly formed in each line configured to have the light emitting elements. The position in a direction orthogonal to the longitudinal direction of the light irradiation unit 11c is disposed such that the center between the blue light emitting elements 3 adjacent to each other is substantially coincident with the center between the red light emitting elements 2.

In the light irradiation unit 11d shown in FIG. 2D, there are three lines formed along the longitudinal direction of the light irradiation unit 11d by the light emitting elements configured to have a plurality of blue light emitting elements 3 and a plurality of red light emitting elements 2. The intervals between the light emitting elements adjacent to each other are regularly formed in each line configured to have the light emitting elements. As shown in FIG. 2D, each line is linearly arranged in a row with a plurality of units formed of a blue light emitting element 3 and two red light emitting elements 2 similarly to the light irradiation unit 11a shown in FIG. 2A. The position in a direction orthogonal to the longitudinal direction of the light irradiation unit 11d is substantially coincident from two lines between which a line located in the center among the three lines is interposed. The position in a direction orthogonal to the longitudinal direction of the light irradiation unit 11d is disposed such that the center between the light emitting elements in the line located in the center is substantially coincident with the center between the light emitting elements in the other two lines. In addition, in the direction orthogonal to the longitudinal direction of the light irradiation unit 11d, the combination of the light emitting elements disposed in the other two lines is set to be a blue light emitting element 3 and a red light emitting element 2 or to be two red light emitting elements 2.

In addition, it is preferable that the light irradiation unit of the present invention have mixed color light emitting elements where the red light emitting elements and the blue light emitting elements are equipped in a package. It is preferable that such mixed color light emitting elements have a function of independently controlling the red light emitting elements and the blue light emitting elements. In addition, as described above, the light emission intensity ratio of the red light emitting element to the blue light emitting element included in mixed light emitting elements (total intensity of red light : total intensity of blue light) is preferably within a range between 2:1 to 9:1, and more preferably within a range between 2:1 to 5:1. It is possible to increase the density of light emitting units in the light irradiation unit using such mixed light emitting elements.

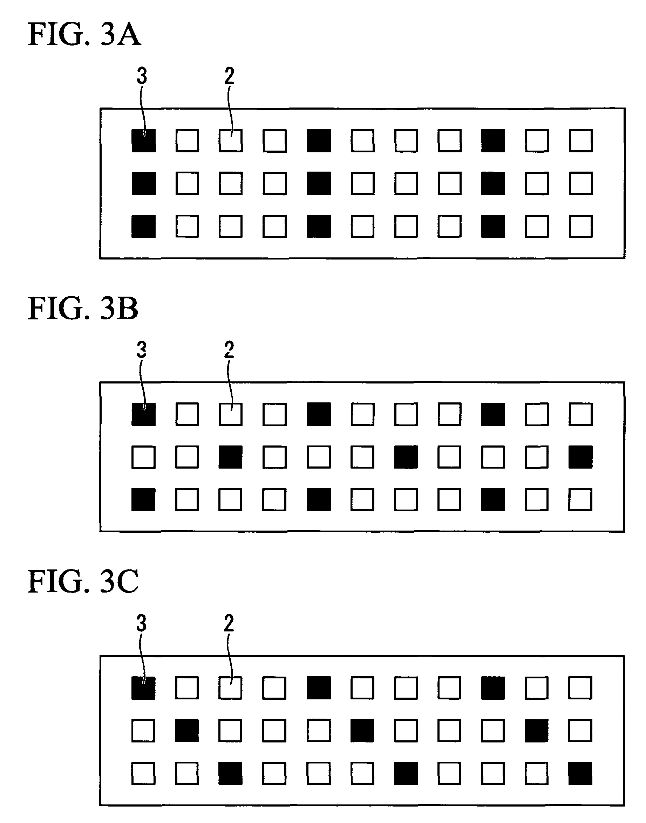

The light irradiation units shown in FIGS. 3A to 3C are ones in which the ratio of the number of red light emitting elements 2 to the number of blue light emitting elements 3 is set as 3:1, and each of the light irradiation units shown in FIGS. 3A to 3 C corresponds to each of those shown in FIGS. 2A, 2B, 2D. Each of FIGS. 3A to 3C show a part of the light irradiation unit, in which all the lines configured to have the light emitting elements are repeatedly arranged with a plurality of units formed of a blue light emitting element 3 and three red light emitting elements 2.

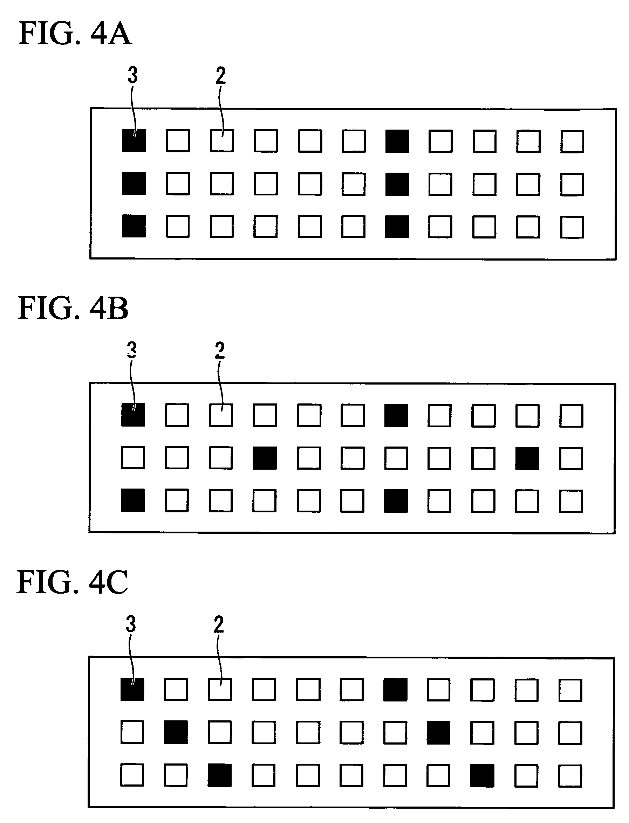

The light irradiation units shown in FIGS. 4A to 4C are ones in which the ratio of the number of red light emitting elements 2 to the number of blue light emitting elements 3 is set as 5:1, and each of the light irradiation units shown in FIGS. 4A to 4 C corresponds to each of those shown in FIGS. 2A, 2B, 2D. Each of FIGS. 4A to 4C show a part of the light irradiation unit, in which all the lines configured to have the light emitting elements are repeatedly arranged with a plurality of units formed of a blue light emitting element 3 and five red light emitting elements 2.

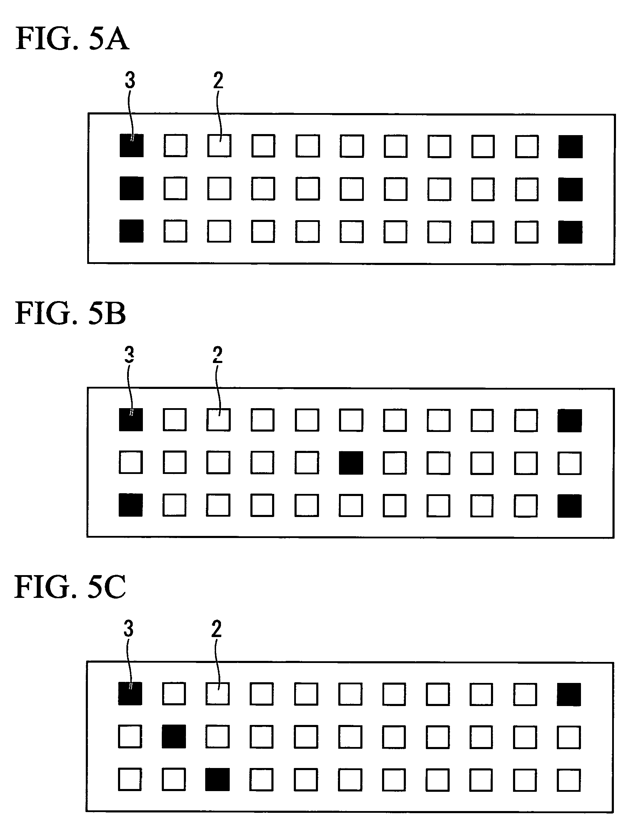

The light irradiation units shown in FIGS. 5A to 5C are ones in which the ratio of the number of red light emitting elements 2 to the number of blue light emitting elements 3 is set as 5:1, and each of the light irradiation units shown in FIGS. 5A to 5C corresponds to each of those shown in FIGS. 2A, 2B, 2D. Each of FIGS. 5A to 5C show a part of the light irradiation unit, in which all the lines configured to have the light emitting elements are repeatedly arranged with a plurality of units formed of a blue light emitting element 3 and nine red light emitting elements 2.

For any case of the ratios of 3:1, 5:1, and 9:1, the red light emitting elements 2 and the blue light emitting elements 3 may be arranged respectively in separate lines, and the lines of the red light emitting elements and the lines of the blue light emitting elements are regularly arranged according to the ratio of the number of the red light emitting elements and the number of the blue light emitting elements, as shown in FIG. 2C.

In the present embodiment, it is possible to use conventionally known ones as red light emitting elements 2 and blue light emitting elements 3. Specifically, for example, it is possible to use a light emitting diode (LED) element, a laser diode (LD) element, electroluminescence (EL) element or the like in which it is easy to select a wavelength and that emits light having a large proportion of light energy of an effective wavelength range. In a case where the EL element is used as the red light emitting elements 2 and the blue light emitting elements 3, an organic EL element may be used or an inorganic EL element may be used.

Examples of red light emitted from the red light emitting elements 2 include light having a wavelength between 570 nm and 730 nm and red light having a wavelength between 645 nm and 680 nm as a center wavelength is suitably used. Examples of blue light emitted from the blue light emitting elements 3 include light having a wavelength between 400 nm and 515 nm and blue light having a wavelength of 450 nm as a center wavelength is suitably used. The red light and the blue light can be set to have a predetermined wavelength range by setting the above-described wavelengths as center wavelengths. Examples of the wavelength range include, in a case of blue light, 450 nm ± 30 nm, preferably 450 nm ± 20 nm, more preferably, 450 nm ± 10 nm.

The light emission intensity of red light and blue light from the light irradiation unit 11 is not particularly limited, but it is preferable to set the light emission intensity ratio of the red light emitting element to the blue light emitting element to be in a range of 1µmol/m²s to 1000 µmol/m²s, preferably 10 µmol/m²s to 500 µmol/m²s, and particularly preferably 50 µmol/m²s to 250 µmol/m²s by the photosynthetic photon flux density (PPFD).

In the present embodiment, it is designed that the light emission intensity of red light and blue light from the light irradiation unit 11 can be controlled such that the amount of electric current supplied to the red light emitting elements 2 or blue light emitting elements 3 is adjusted by the control unit provided in the plant cultivation lamp 1.

One end of the light irradiation unit 11 in the plant cultivation lamp 1 shown in FIG. 1 is provided with two input terminals 41 and 43 for the red light emitting elements 2 and the other end thereof is provided with two input terminals 42 and 44 for the blue light emitting elements 3.

A plurality of red light emitting elements 2 is electrically connected to input terminals 41 and 43 for the red light emitting elements 2 via a wire (not shown).

A plurality of blue light emitting elements 3 is electrically connected to input terminals 42 and 44 for the blue light emitting elements 3 via a wire (not shown).

The control unit provided in the plant cultivation lamp 1 of the present embodiment independently turns on and off the red light emitting elements 2 and the blue light emitting elements 3 by supplying a predetermined electric current to the red light emitting elements 2 and the blue light emitting elements 3 via the input terminals 41 and 43 for the red light emitting elements 2 or input terminals 42 and 44 for the blue light emitting elements 3.

In the present embodiment, the control unit is provided with a lamp controller (light emission intensity control means) that controls the light emission intensity ratio of the red light to the blue light emitted from the light irradiation unit 11.

Examples of the lamp controller include a lamp controller that controls the light emission intensity ratio of the red light to the blue light omitted from the light irradiation unit 11 by adjusting the amount of electric current supplied to the red light emitting elements 2 or blue light emitting elements 3 and by changing the intensity ratio of part or all of red light emitting elements 2 and/or part or all of blue light emitting elements 3, or a lamp controller that controls the number of red light emitting elements 2 and/or blue light emitting elements 3 to be turned on and controls the light emission intensity ratio of the red light to the blue light emitted from the light irradiation unit 11 by supplying a predetermined electric current to a part of red light emitting elements 2 and/or blue light emitting elements 3.

Specifically, for example, the plant cultivation lamp 1 shown in FIG. 1 may be a plant cultivation lamp that emits light in which the light emission intensity of blue light is the same as the light emission intensity of the red light, from the light irradiation unit 11 by supplying an identical electric current to all of the red light emitting elements 2 and all of the blue light emitting elements 3 using the lamp controller. In a case where an identical electric current is supplied to all of the red light emitting elements 2 and all of the blue light emitting elements 3, the plant cultivation lamp 1 may not be provided with the lamp controller.

In addition, the plant cultivation lamp 1 shown in FIG. 1 may be a plant cultivation lamp that emits light in which the light emission intensity ratio of the red light to the blue light is 2:1 (red light : blue light), from the light irradiation unit 11 by setting the light emission intensity of the blue light emitting elements 3 to be half of the light emission intensity of the red light emitting elements 2 using the lamp controller, for example.

In addition, when using the lamps for plant cultivation shown in FIGS. 2A to 2D, a used plant cultivation lamp may output emitted light in which the light emission intensity ratio of the red light to the blue light is 2:1 (red light : blue light), from the light irradiation unit by supplying an identical electric current to all of the red light emitting elements 2 and all of the blue light emitting elements 3 using the lamp controller, for example. In this case, since an identical electric current is supplied to all of the red light emitting elements 2 and all of the blue light emitting elements 3, the plant cultivation lamp may not be provided with the lamp controller.

In addition, the lamps for plant cultivation 1 shown in FIGS. 2A to 2D may be lamps for plant cultivation that emit emitted light in which the light emission intensity ratio of red light to blue light is 4:1 (red light: blue light), from the light irradiation unit by setting the light emission intensity of the blue light emitting elements 3 to be half of the light emission intensity of the red light emitting elements 2 using the lamp controller, for example.

The light emission intensity ratio of the red light to the blue light from the light irradiation unit 11 (red light : blue light) is preferably within a range between 2:1 to 9:1, and more preferably within a range between 2:1 to 5:1. In a case where the light emission intensity ratio of the red light to the blue light is within the above-described range, it is possible to obtain a sufficient effect of promoting the growth of a plant, the effect being caused by making the light emission intensity of red light higher than the light emission intensity of blue light. In a case where the light emission intensity ratio of red light to blue light is lower than the above-described range, there is a concern that it is impossible to obtain a sufficient effect of promoting the growth of a plant, the effect being caused by making the light emission intensity of red light higher than the light emission intensity of blue light because it is impossible to obtain a sufficient difference between the light emission intensity of the red light and the light emission intensity of the blue light. In a case where the light emission intensity ratio of red light to blue light is higher than the above-described range, there is a concern that it is impossible to obtain sufficient effect of promoting the growth of a plant because the light emission intensity of red light is too high.

### [Plant cultivation method]

Next, as the plant cultivation method of the present embodiment, a method of cultivating a plant using the plant cultivation lamp 1 shown in FIG. 1 will be described as an example.

The plant cultivation method of the present embodiment includes a step of independently performing a sequence of irradiating a plant with red light (hereinafter, referred to as "red light irradiation step) and a sequence of irradiating a plant with blue light (hereinafter, referred to as "blue light irradiation step) within a certain period of time (Shigyo method).

Here, the "certain period of time" meansan arbitrary period of time during plant cultivation. The arbitrary period is the full length of the whole cultivation period at longest. In addition, the shortest period can be arbitrarily set as long as the present invention exhibits an effect. In this period, the unit of the length of time is between 3 hours and 48 hours. Moreover, flashing irradiation with a high frequency of greater than or equal to 1 Hz is not included in the "certain period of time" of the present invention.

In addition, the term "independently" means that there is a red light irradiation step and a blue light irradiation step separately within the period described above.

At least one process of the red light irradiation step and at least one process of the blue light irradiation step are included within the period described above. The red light irradiation step and the blue light irradiation step may be alternatingly performed in a continuous manner or may be discontinuously repeated by having a sequence of simultaneously irradiating a plant with red light and blue light or a sequence of suspending light irradiation on a plant, each sequence being interposed between both steps.

In the whole period of time of plant cultivation immediately after germinating seeds or immediately after setting out seedlings until harvest, the plant cultivation method according to the present invention can start or finish at an arbitrary timing and can be applied for a length of arbitrary time.

Plants cultivated using the plant cultivation method according to the present invention are not particularly limited, and examples thereof include vegetables, potatoes, mushrooms, fruits, pulses, grains, nuts, algae, ornamental plants, and mosses.

In the plant cultivation method of the present embodiment, since the Shigyo method is performed using the plant cultivation lamp 1 of the present embodiment, it is possible to easily irradiate a plant to be grown with optimum artificial light thereby obtaining an excellent effect of promoting the growth of a plant.

In addition, in the plant cultivation method of the present embodiment, it is possible to set the light emission intensity ratio of red light to blue light emitted from the light irradiation unit 11 to be more suitable for raising a plant depending on the type of plant to be cultivated using a plant cultivation lamp in which the control unit is provided with a lamp controller (light emission intensity control means) that controls the light emission intensity ratio of red light to blue light emitted from the light irradiation unit 11, as the plant cultivation lamp 1.

The plant cultivation lamp 1 of the present embodiment includes a control unit that independently turns on and off the red light emitting elements 2 and the blue light emitting elements 3. Therefore, it is possible to simultaneously or alternatingly irradiate a plant with red light and blue light or to change the duration of the irradiation with red light and blue light depending on the plant to be grown so as to obtain a sufficient effect of promoting the growth of the plant using the Shigyo method, thereby obtaining an excellent effect of promoting the growth of the plant.

In addition, the plant cultivation lamp 1 of the present embodiment includes the light irradiation unit having the red light emitting elements 2 and the blue light emitting elements 3. Therefore, it is possible to easily secure a space used to dispose the irradiation means and to reduce the deviation between the direction from which a plant is irradiated with red light and the direction from which a plant is irradiated with blue light in comparison with a case where two types of irradiation means, a means of irradiating red light and a means of irradiating blue light, are disposed in the lamp.

In addition, in a case where the control unit of the plant cultivation lamp 1 of the present embodiment includes the lamp controller used to control the light emission intensity ratio of red light to blue light emitted from the light irradiation unit 11, it is possible to easily change the intensity ratio of red light to blue light, thereby easily providing an optimum intensity ratio to a plant to be grown.

In addition, the plant cultivation lamp 1 of the present embodiment includes a light irradiation unit 11 having a long rectangular shape in planar view. Therefore, it is possible to substitute the light irradiation unit with lighting equipment such as a straight tube fluorescent lamp of the related art, and the light irradiation unit thereby may be easily installed in a position in which lighting equipment of the related art is installed.

In addition, it is preferable that the light irradiation unit have a red conversion means electrically connected to red light emitting elements and a blue conversion means electrically connected to blue light emitting elements. The red conversion means outputs AC power, which is input, by converting the AC power into DC power and has a function used to control an output electric current which is supplied to the red light emitting element. In addition the blue conversion means outputs AC power, which is input, by converting the AC power into DC power and has a function used to control an output electric current which is supplied to the blue light emitting elements.

In a case where the light irradiation unit includes the red conversion means and the blue conversion means, it is possible to input the AC power to the light irradiation unit and to supply the DC power to the red light emitting elements and the blue light emitting elements. Accordingly, it is possible to install the plant cultivation lamp of the present invention similarly to the fluorescent lamp in the related art. In addition, in a case where the light irradiation unit includes the red conversion means and the conversion means for blue, it is possible to effectively utilize the space compared to a case where conversion means for converting the AC power into the DC power is separately installed from the plant cultivation lamp, which is desirable.

Furthermore, in a case where the light irradiation unit has a long rectangular shape in planar view and includes red conversion means and conversion means for blue, it is preferable that one end of the light irradiation unit be provided with two input terminals for red light emitting elements electrically connected to the red conversion means and the other end be provided with two input terminals for the blue light emitting elements electrically connected to the conversion means for blue. In this case, it is possible to easily dispose the respective red conversion means and conversion means for blue in both ends of the light irradiation unit and to easily disperse the red conversion means and the blue conversion means as heat generation sources, thereby obtaining a plant cultivation lamp excellent in heat radiation.

In addition, when cultivating a plant using the plant cultivation lamp of the present invention, in general, a plurality of lamps for plant cultivation are simultaneously used. When simultaneously using a plurality of lamps for plant cultivation, it is preferable to use a light dimmer system that can control the light emission intensity of the plurality of lamps for plant cultivation. Examples of the light dimmer systems include a light dimmer system that separately controls the power supplied to each plant cultivation lamp.

The present invention is not limited to the above-described embodiment.

For example, in the above-described embodiment, an example of a plant cultivation lamp in which the control unit includes light emission intensity control means as the plant cultivation lamp of the present invention is described, but the control unit may not include the lamp controller. In this case, it is possible to reduce the number of members used in the plant cultivation lamp in comparison to a plant cultivation lamp provided with the lamp controller, which is preferable.

### [Example]

In the following Example, leaf lettuce (variety: Summer surge) was used as an objective plant to observe a growth state. First, 6 seeds of the leaf lettuce plants were sown in a peat plate used for growing at regular intervals and were germinated under a fluorescent light (12 hour day length). All the test groups were placed under an identical light environment for 3 days from sowing until germination.

Thereafter, the leaf lettuce plants were placed in meteorological instruments under different light environments of Examples 1 to 3, and then were grown for 24 hours. All the environments within the meteorological instruments used in Examples 1 to 3 were set to identical conditions at 25°C to 27°C of the atmospheric temperature and 60% of humidity, except for the light irradiation condition.

### (Example 1)

In Example 1, a plant cultivation lamp that includes a light irradiation unit having red light emitting elements formed of 240 red LEDs (center wavelength: 660 nm, HRP-350F made by SHOWA DENKO K.K.) and having blue light emitting elements formed of 240 blue LEDs (center wavelength: 450 nm, GM2LR450G made by SHOWA DENKO K.K.), and a control unit that controls the light irradiation unit to independently turn on and off the red light emitting elements and the blue light emitting elements was used.

In the light irradiation unit, red light emitting elements and blue light emitting elements are arranged as shown in FIG. 1.

The photosynthetic photon flux density (PPFD) as a light emission intensity of red light from the light irradiation unit was set as 150 µmol/m²s in total and the photosynthetic photon flux density (PPFD) as a light emission intensity of blue light from the light irradiation unit was set as 150 µmol/m²s in total (light emission intensity ratio of red light to blue light is 1:1).

Moreover, a red light irradiation step of irradiating a plant only with red light and a blue light irradiation step of irradiating a plant only with blue light were separately performed in a continuous manner for 1 day, being 12 hours per each color. There was no time where irradiation was not performed using any light.

### (Example 2)

The same test as Example 1 was performed except that a plant cultivation lamp was used that includes a light irradiation unit having red light emitting elements formed of 320 red LEDs (center wavelength: 660 nm, HRP-350F made by SHOWA DENKO K.K.) and having blue light emitting elements formed of 160 blue LEDs (center wavelength: 450 nm, GM2LR450G made by SHOWA DENKO K.K.).

In the light irradiation unit, red light emitting elements and blue light emitting elements are arranged as shown in FIG. 2A.

The photosynthetic photon flux density (PPFD) of red light from the light irradiation unit was set as 200 µmol/m²s and the photosynthetic photon flux density (PPFD) of blue light from the light irradiation unit was set as 100 µmol/m²s (light emission intensity ratio of red light to blue light is 2:1).

### (Example 3)

The same test as Example 1 was performed except for the use of the same light irradiation unit as that in Example 2 and a plant cultivation lamp that has a control unit provided with a lamp controller (light emission intensity control means) which can be used to change the light emission intensity ratio of red light to blue light by the lamp controller.

The photosynthetic photon flux density (PPFD) of red light as the light emission intensity of red light from the light irradiation unit was set to 225 µmol/m²s and the photosynthetic photon flux density (PPFD) of blue light from the light irradiation unit was set to 75 µmol/m²s (light emission intensity ratio of red light to blue light is 3:1).

### (Example 4)

The same test as Example 1 was performed except for the use of the same light irradiation unit as that in Example 2 and a plant cultivation lamp that has a control unit provided with a lamp controller (light emission intensity control means) which can be used to change the light emission intensity ratio of red light to blue light by the lamp controller.

The photosynthetic photon flux density (PPFD) of red light as the light emission intensity of red light from the light irradiation unit was set to 250 µmol/m²s and the photosynthetic photon flux density (PPFD) of blue light from the light irradiation unit was set to 50 µmol/m²s (light emission intensity ratio of red light to blue light is 5:1).

### (Example 5)

The same test as Example 1 was performed except for the use of a light irradiation unit as shown in FIG. 5B, in which the ratio of the number of the red light emitting elements and the number of the blue light emitting elements is 9:1.

The photosynthetic photon flux density (PPFD) of red light from the light irradiation unit was set as 270 µmol/m²s and the photosynthetic photon flux density (PPFD) of blue light from the light irradiation unit was set as 30 µmol/m²s (light emission intensity ratio of red light to blue light is 9:1).

### (Comparative Example)

The light emission intensity ratio of red light to blue light was set as 1:1 and light including the red light and the blue light was turned on for 12 hours and turned off for 12 hours during a day using a lamp in which the red light and the blue light were not separated so as to be under the same light conditions as Example 1.

As to the leaf lettuces grown for 24 days in Examples 1 to 3 and Comparative Example, the weight of above-ground part (above-ground part fresh weight) was measured to obtain a mass ratio by setting the mass ratio of Example 1 as 100%. The result is shown in Table 1.

**TABLE 1**

| | INTENSITY RATIO OF RED LIGHT TO BLUE LIGHT | FRESH WEIGHT RATIO OF ABOVE-GROUND PART (%) |
|---|---|---|
| EXAMPLE1 | 1:1 | 100 |
| EXAMPLE2 | 2:1 | 140 |
| EXAMPLE3 | 3:1 | 160 |
| EXAMPLE4 | 5:1 | 145 |
| EXAMPLE5 | 9:1 | 110 |
| COMPARATIVE EXAMPLE | | 70 |

As shown in Table 1, it was found that Example 2 in which a plant was irradiated with artificial light having the light emission intensity ratio of the red light to blue light of 2:1 exhibits a high effect of promoting growth of a plant in comparison with Example 1 in which a plant was irradiated with artificial light having a light emission intensity ratio of red light to blue light of 1:1. Furthermore, it was found that Example 3 in which a plant was irradiated with artificial light having a light emission intensity ratio of red light to blue light of 3:1 exhibits a high effect of promoting growth of a plant in comparison with Example 2. Furthermore, it was found that Example 4 in which a plant was irradiated with artificial light having a light emission intensity ratio of red light to blue light of 5:1 exhibits a high effect of promoting growth of a plant in comparison with Example 2, and Example 4 exhibits a low effect of promoting growth of a plant in comparison with Example 3. Furthermore, it was found that Example 5 in which a plant was irradiated with artificial light having a light emission intensity ratio of red light to blue light of 9:1 exhibits a high effect of promoting growth of a plant in comparison with Example 1, and Example 5 exhibits a low effect of promoting growth of a plant in comparison with Examples 2 to 4.

In addition, it was found that Comparative Example in which a plant was irradiated with light including the red light and the blue light exhibits a low effect for promoting growth of a plant in comparison with Example 1 because the above-ground part fresh weight ratio in Comparative Example is 70%.

As plants which shows the same results described above, for example, there are perilla, leeks, Japanese chervil, and watercress, sprouted Welsh onion, tomatoes, strawberries, melons, green peppers, and okra, other than leaf lettuce.

While preferred embodiments of the invention have been described and illustrated above, it should be understood that these are exemplary examples of the invention and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims.

## Claims

1. A plant cultivation lamp, comprising:
a light irradiation unit that includes one or more red light emitting elements that emit red light and one or more blue light emitting elements that emit blue light; and
a control unit that controls the light irradiation unit to independently turn on and off the red light emitting elements and the blue light emitting elements so as to alternately perform red light irradiation for between 3 hours and 48 hours and blue light irradiation for between 3 hours and 48 hours in a continuous manner.

2. The plant cultivation lamp according to Claim 1,
wherein the light irradiation unit includes mixed color light emitting elements where the red light emitting elements and the blue light emitting elements are equipped with an identical package.

3. The plant cultivation lamp according to Claim 1 or 2,
wherein the light emission intensity ratio of the red light emitting element to the blue light emitting element is in a range between 2:1 and 9:1.

4. The plant cultivation lamp according to any one of Claim 1 to 3,
the light irradiation unit includes plural identical red light emitting elements and plural identical blue light emitting elements,
wherein the ratio of the number of the red light emitting elements and the number of the blue light emitting elements is in the range of 2:1 to 9:1,
wherein the red light emitting elements and the blue light emitting elements are arranged to be regularly mixed.

5. The plant cultivation lamp according to any one of Claims 1 to 4,
wherein the control unit includes light emission intensity control means for controling the light emission intensity ratio of red light to blue light emitted from the light irradiation unit.

6. The plant cultivation lamp according to any one of Claims 1 to 5,
wherein the light irradiation unit has a long rectangular shape in planar view and has an outer shape compatible with a customized straight tube fluorescent lamp.

7. The plant cultivation lamp according to any one of Claims 1 to 6,
wherein the light irradiation unit includes red conversion means for red which is electrically connected to the red light emitting elements and conversion means for blue which is electrically connected to the blue light emitting elements,
wherein the red conversion means for red outputs DC power converted from AC power which is input and has a function to control an output electric current which is supplied to the red light emitting element, and
wherein the conversion means for blue outputs DC power converted from AC power which is input and has a function to control an output electric current which is supplied to the blue light emitting elements.

8. The plant cultivation lamp according to Claim 7,
wherein the light irradiation unit has a long rectangular shape in planar view and includes two input terminals for the red light emitting elements electrically connected to the red conversion means in one end and two input terminals for the blue light emitting elements electrically connected to the conversion means for blue in the other end.

9. A plant cultivation method using a plant cultivation lamp according to any one of Claims 1 to 8, comprising:
a sequence of irradiating a plant with red light; and
a sequence of irradiating a plant with blue light,
wherein the sequences are independently and alternately performed within a certain period of time, wherein the irradiation with red light is performed in a continuous manner for between 3 hours and 48 hours, and the irradiation with blue light is performed in a continuous manner for between 3 hours and 48 hours.

## Patentansprüche

1. Pflanzenkultivierungslampe, welche umfasst:
eine Lichtstrahlungseinheit, die ein oder mehrere rotes Licht emittierende Rotlichtemissionselemente und ein oder mehrere blaues Licht emittierende Blaulichtemissionselemente umfasst; und
eine Kontrolleinheit, welche die Lichtstrahlungseinheit dahingehend kontrolliert, dass die Rotlichtemissionselemente und die Blaulichtemissionselemente unabhängig voneinander ein- und ausgeschaltet werden, so dass abwechselnd Rotlichtbestrahlung zwischen 3 Stunden und 48 Stunden und Blaulichtbestrahlung zwischen 3 Stunden und 48 Stunden auf kontinuierliche Weise durchgeführt wird.

2. Pflanzenkultivierungslampe nach Anspruch 1,
wobei die Lichtstrahlungseinheit Mischfarbenlichtemissionselemente umfasst, wobei die Rotlichtemissionselemente und die Blaulichtemissionselemente mit einer identischen Einheit ausgestattet sind.

3. Pflanzenkultivierungslampe nach Anspruch 1 oder 2,
wobei das Lichtemissionsintensitätsverhältnis des Rotlichtemissionselements zu dem Blaulichtemissionselement im Bereich zwischen 2:1 und 9:1 liegt.

4. Pflanzenkultivierungslampe nach einem der Ansprüche 1 bis 3,
wobei die Lichtstrahlungseinheit eine Vielzahl von identischen Rotlichtemissionselementen und eine Vielzahl von identischen Blaulichtemissionselementen enthält,
wobei das Verhältnis der Anzahl der Rotlichtemissions-elemente zu der Anzahl der Blaulichtemissionselemente im Bereich von 2:1 bis 9:1 liegt,
wobei die Rotlichtemissionselemente und die Blaulichtemissionselemente so angeordnet sind, dass sie gleichmäßig gemischt sind.

5. Pflanzenkultivierungslampe nach einem der Ansprüche 1 bis 4,
wobei die Kontrolleinheit Lichtemissionsintensitätskontrollmittel zum Kontrollieren des Lichtemissionsintensitätsverhältnisses von rotem Licht zu blauem Licht, das von der Lichtstrahlungseinheit emittiert wird, umfasst.

6. Pflanzenkultivierungslampe nach einem der Ansprüche 1 bis 5,
wobei die Lichtstrahlungseinheit eine lange rechtwinklige Form in der Draufsicht hat und eine äußere Form aufweist, die mit einer angepassten Geradrohrfluoreszenzlampe kompatibel ist.

7. Pflanzenkultivierungslampe nach einem der Ansprüche 1 bis 6,
wobei die Lichtstrahlungseinheit Rotkonversionsmittel für Rot enthält, die mit den Rotlichtemissionselementen elektrisch verbunden sind, und Konversionsmittel für blaues Licht enthält, die mit den Blaulichtemissionselementen elektrisch verbunden sind,
wobei die Rotkonversionsmittel für Rot von Wechselstrom umgewandelten Gleichstrom erzeugt und die Funktion hat, den ausströmenden elektrischen Strom, der dem Rotlichtemissionselement zugeführt wird, zu kontrollieren, und
wobei die Konversionsmittel für Blau von Wechselstrom umgewandelten Gleichstrom erzeugen und die Funktion haben, den ausströmenden elektrischen Strom, der den Blaulichtemissionselementen zugeführt wird, zu kontrollieren.

8. Pflanzenkultivierungslampe nach Anspruch 7,
wobei die Lichtstrahlungseinheit eine lange rechtwinklige Form in der Draufsicht aufweist und zwei Eingangsanschlüsse für die Rotlichtemissionselemente, die an einem Ende mit den Rotkonversionsmitteln elektrisch verbunden sind, und zwei Einlassanschlüsse für die Blaulichtemissionselemente umfasst, die mit den Konversionsmitteln für Blau am anderen Ende elektrisch verbunden sind.

9. Pflanzenkultivierungsverfahren unter Einsatz einer Pflanzenkultivierungslampe nach einem der Ansprüche 1 bis 8, welches umfasst:
eine Sequenz der Belichtung einer Pflanze mit rotem Licht; und
eine Sequenz der Belichtung einer Pflanze mit blauem Licht,
wobei die Sequenzen unabhängig voneinander und abwechselnd innerhalb eines bestimmten Zeitraums durchgeführt werden, wobei die Belichtung mit rotem Licht zwischen 3 Stunden und 48 Stunden kontinuierlich durchgeführt wird und die Belichtung mit blauem Licht zwischen 3 Stunden und 48 Stunden kontinuierlich durchgeführt wird.

## Revendications

1. Lampe de culture de plantes, comprenant:
une unité d'irradiation de lumière comprenant un ou plusieurs élément(s) d'émission de lumière rouge qui émet(tent) une lumière rouge, un ou plusieurs élément(s) d'émission de lumière bleue qui émet(tent) une lumière bleue; et
une unité de commande qui commande l'unité d'irradiation de lumière de manière à activer et désactiver de façon indépendante les éléments d'émission de lumière rouge et les éléments d'émission de lumière bleue de manière à exécuter alternativement une irradiation de lumière rouge pendant entre 3 heures et 48 heures et une irradiation de lumière bleue pendant entre 3 heures et 48 heures d'une manière continue.

2. Lampe de culture de plantes selon la revendication 1,
dans laquelle l'unité d'irradiation de lumière comprend des éléments d'émission de lumière de couleur mixte dans lesquels les éléments d'émission de lumière rouge et les éléments d'émission de lumière bleue sont équipés d'un emballage identique.

3. Lampe de culture de plantes selon la revendication 1 ou 2,
dans laquelle le rapport entre l'intensité d'émission de lumière de l'élément d'émission de lumière rouge et l'intensité d'émission de lumière de l'élément d'émission de lumière bleue est compris dans une gamme de 2:1 à 9:1.

4. Lampe de culture de plantes selon l'une quelconque des revendications 1 à 3,
dans laquelle l'unité d'irradiation de lumière comprend plusieurs éléments d'émission de lumière rouge identiques et plusieurs éléments d'émission de lumière bleue identiques,
dans laquelle le rapport entre le nombre d'éléments d'émission de lumière rouge et le nombre d'éléments d'émission de lumière bleue est compris dans la gamme de 2:1 à 9:1,
dans laquelle les éléments d'émission de lumière rouge et les éléments d'émission de lumière bleue sont agencés de manière à être mélangés de façon régulière.

5. Lampe de culture de plantes selon l'une quelconque des revendications 1 à 4,
dans laquelle l'unité de commande comprend des moyens de commande d'intensité d'émission de lumière pour commander le rapport d'intensité d'émission de lumière entre la lumière rouge et la lumière bleue émises par l'unité d'irradiation de lumière.

6. Lampe de culture de plantes selon l'une quelconque des revendications 1 à 5,
dans laquelle l'unité d'irradiation de lumière présente une forme rectangulaire longue dans une vue en plan et présente une forme extérieure compatible avec une lampe fluorescente à tube droit personnalisée.

7. Lampe de culture de plantes selon l'une quelconque des revendications 1 à 6,
dans laquelle l'unité d'irradiation de lumière comprend un moyen de conversion de rouge pour le rouge qui est électriquement connecté aux éléments d'émission de lumière rouge, et un moyen de conversion pour le bleu qui est électriquement connecté aux éléments d'émission de lumière bleue,
dans laquelle le moyen de conversion de rouge pour le rouge génère du courant continu converti en courant alternatif qui est entré et a pour fonction de commander un courant électrique de sortie qui est fourni aux éléments d'émission de lumière rouge, et
dans laquelle le moyen de conversion pour le bleu génère du courant continu converti en courant alternatif qui est entré et a pour fonction de commander un courant électrique de sortie qui est fourni aux éléments d'émission de lumière bleue.

8. Lampe de culture de plantes selon la revendication 7,
dans laquelle l'unité d'irradiation de lumière présente une forme rectangulaire dans une vue en plan et comprend deux bornes d'entrée pour les éléments d'émission de lumière rouge électriquement connectées aux moyens de conversion de rouge à une première extrémité, et deux bornes d'entrée pour les éléments d'émission de lumière bleue électriquement connectées aux moyens de conversion pour le bleu à l'autre extrémité.

9. Procédé de culture de plantes utilisant une lampe de culture de plantes selon l'une quelconque des revendications 1 à 8, comprenant:
une séquence d'irradiation d'une plante avec une lumière rouge; et
une séquence d'irradiation d'une plante avec une lumière bleue,
dans laquelle les séquences sont exécutées de façon indépendante et alternative à l'intérieur d'une certaine période de temps, dans lequel l'irradiation avec une lumière rouge est exécutée d'une manière continue pendant entre 3 heures et 48 heures, et l'irradiation avec une lumière bleue est exécutée d'une manière continue pendant entre 3 heures et 48 heures.
